# EUROPÄISCHE PATENTANMELDUNG

(11) **EP 1 366 721 A1**
(43) Veröffentlichungstag der Anmeldung: **03.12.2003**
(21) Anmeldenummer: 03002922.7
(22) Anmeldetag: 10.02.2003
(51) Int. Cl.: A61B 17/16, A61B 17/17, A61F 2/46

(54) **Vorrichtung zur Vorbereitung eines Femurknochens für die Implantation einer Prothese**

(30) Priorität: 23.05.2002 FR 0206291
(71) Anmelder: Centerpulse Orthopedics Ltd., 6340 Baar (CH)
(72) Erfinder: Le Beguec, Pierre, 35000 Rennes (FR)
(74) Vertreter: Manitz, Finsterwald & Partner GbR

(57) **Zusammenfassung**

Vorrichtung zur Vorbereitung eines Femurknochens für die zementfreie Implantation einer Prothese mit sog. Press-fit-Effekt, dadurch gekennzeichnet, daß sie einerseits aus einer modulierbaren Raspel (10) aus mehreren Teilen besteht, und zwar einem distalen Teil (10A) und mindestens zwei anderen proximalen Teilen (10B) von wesentlich verschiedenen Formen (a,b) und von aufsteigenden Höhen (H), die gemaß einer in Abhängigkeit vom Morphotyp und vom Grad der Versenkung des distalen Teils (10A) bestimmten Wahl mit dem distalen Teil (10A) durch lösbare Verbindungsmittel (11) fest verbunden werden können, und andererseits aus einem neutralen Zwischenelement (12), das eine proximale Lehre bildet, die für die vorausgehende Anbringung des distalen Teils (10A) der Raspel (10) und für die Messung seiner Versenkung an einer Gradteilung (13) bestimmt ist, die die richtige Länge des wiederherzustellenden Femur und damit die Große des zu wählenden proximalen Teils (10B) der Raspel (10) angibt.

## Beschreibung

Die Erfindung betrifft die Vorbereitung eines Femurknochens zum Zweck der zementfreien Implantation einer Prothese mit sogenanntem Press-fit-Effekt.

Im Bereich der Hüftprothesen sind zwei große Gruppen von zementfreien Femurschäften bekannt, die dazu bestimmt sind, in einem Femurknochen mit Hilfe einer Ahle oder einer Raspel implantiert zu werden.

Es gibt einerseits sog. Erstimplantations-Femurprothesen, die am Patienten zum ersten Mal eingesetzt werden, und andererseits sog. Revisionsprothesen, die, wie der Name schon sagt, ein Implantat, das versagt hat, ersetzen können.

In beiden Fällen besteht das Hauptziel darin, eine vollkommene Primärstabilität des Femurschaftes oder Implantats zu gewährleisten.

Um dieses Ziel zu erreichen, d.h. eine feste mechanische Verbindung zwischen der Prothese und dem umgebenden Knochen herzustellen, gibt es mehrere Verfahren, von denen zwei häufig angewandt werden, und zwar die Verwendung eines verriegelten Schafts und das bereits erwähnte sog. Press-fit-Konzept.

Damit das Press-fit-Konzept wirksam ist, müssen zwei wesentliche Bedingungen gleichzeitig erfüllt werden, und zwar die Herstellung einer Kontaktfläche zwischen dem Knochen und dem Implantat und eine einwandfreie Positionierung der Prothese in diesem Kontaktbereich.

Um diese beiden Ziele zu erreichen, muß also eine sorgfältige Vorbereitung des Medullarhohlraums des Femur oder Femurknochens vorgenommen werden, was immer schwierig ist, insbesondere dann, wenn es sich um eine Prothese handelt, und zwar infolge der anatomischen Schwankungen und/oder, wenn dies nicht der Fall ist, infolge der Knochensubstanzverluste.

Die Aufgabe besteht also darin, daß man im Voraus den Bereich des Femur mit Präzision festlegt, in dem man den Press-fit durchführen kann, wobei man im Übrigen weiß, daß in allen Fällen die Längengleichheit der beiden unteren Gliedmaßen des Patienten einzuhalten ist und, wenn es möglich ist, der Bereich des Kontakts Knochen/Implantat auf den proximalen Teil des Femur zu begrenzen ist, um nicht den gesamten Femur zu sehr zu versteifen.

Eine andere Schwierigkeit ergibt sich daraus, daß der sog. Press-fit-Bereich im Femurknochen auf einer unterschiedlichen.Höhe gelegen ist.

Er liegt nämlich selten im oberen metaphysären Bereich, d.h. über dem kleinen Trochanter, da in dieser Höhe zahlreiche anatomische Schwankungen (wenn es sich um ein Erstimplantat handelt) oder Knochensubstanzverluste (wenn es sich um einen Austausch handelt) auftreten.

Der Bereich des Femur, der am sichersten und am besten verwendbar ist, liegt also meistens im mittleren Teil des Femur, d.h. im mataphyso-diaphysären Bereich, d.h. auf Höhe des und unterhalb des kleinen Trochanter in der Zone, die oberhalb des diaphysären Bereichs gelegen ist, wo eine zu starke Befestigung möglichst zu vermeiden ist.

Dieser ideale Bereich für die Durchführung des Press-fit kann also je nach Morphotyp und Ausmaß der Knochenschäden bezüglich der Höhe variieren.

In einer ersten Phase der Erfindung strebte man eine Lösung des Problems an, indem man ein modulierbares System zur Vorbereitung des Femur suchte, das eine abgestufte Vorbereitung des Femur gestattet, was bedeutet, in einem ersten Schritt das Primärstabilitätsbereich genannte Press-fit-Gebiet zu finden und vorzubereiten und in einem zweiten Schritt den Femur in seinem proximalen Bereich vorzubereiten, um den Femurschaft zu wählen, der zur Wiederherstellung der richtigen Länge der unteren Gliedmaße am besten angepaßt ist.

Zu diesem Zweck besteht die Lösung für das gestellte technische Problem und das gesuchte Ziel erfindungsgemäß darin, daß die Vorrichtung zur Vorbereitung eines Femurknochens für die zementfreie Implantation einer Prothese mit sog. Press-fit-Effekt einerseits aus einer modulierbaren Raspel aus mehreren Teilen besteht, von denen ein stark konisches distales Teil von einer einzigen Größe und von einer einzigen Form ist und von denen mindestens zwei andere proximale Teile von verschiedenen Formen und von aufsteigenden Höhen H sind und gemäß einer in Abhängigkeit vom Morphotyp und vom Grad der Versenkung des distalen Teils bestimmten Wahl mit dem distalen Teil durch lösbare Verbindungsmittel fest verbunden werden können, und andererseits aus einem neutralen Zwischenelement, das eine proximale Lehre bildet, die für die vorausgehende Anbringung des distalen Teils der Raspel und für die Messung seiner Versenkung an einer Gradteilung bestimmt ist, die die richtige Länge des wiederherzustellenden Femur und damit die Größe des proximalen Teils der Raspel angibt, das zu wählen und nacheinander an Ort und Stelle des Zwischenelements zur Vorbereitung dieses Femurknochens anzuordnen ist.

Eine solche erfindungsgemäße Vorrichtung besitzt entscheidende Vorteile, und zwar:
- einerseits wird eine gute Vorbereitung des Wahlbereichs gewährleistet, in dem die Primärstabilität im metaphyso-diaphysären Bereich stattfinden soll, und zwar mit dem distalen Teil der Raspel. Die Lehre hat in der Tat keine den Femur vorbereitende Wirkung, sie dient nur zur Messung der wünschenswerten Größe der distalen Raspel,
- andererseits wird eine Blockierung an drei Punkten (im proximalen metaphysären Bereich) vermieden, durch die die Gefahr bestünde, daß es zu einem sekundären Einsinken kommt und zu einer unzureichenden Rotationsstabilität, was mit einer herkömmlichen einstückigen Raspel immer möglich ist und was unbedingt zu vermeiden ist, da es sich dann nicht mehr um einen Press-fit-Effekt, sondern um eine einfache unsichere Blockierung handelt.

Die vorliegende Erfindung betrifft auch die Merkmale, die sich aus der folgenden Beschreibung ergeben und die einzeln oder in allen ihren möglichen technischen Kombinationen zu betrachten sind.

In dieser Beschreibung wird unter Bezugnahme auf die beiliegende Zeichnung als nicht begrenzendes Beispiel beschrieben, wie die Erfindung ausgeführt sein kann. In dieser Zeichnung zeigen:
- Fig. 1: und 2 Schnitte durch Femurknochen von verschiedenen Morphotypen mit Press-fit-Gebieten mit ebenfalls verschiedenen Höhen,
- Fig. 3: einen Schnitt durch einen Femurknochen gemäß einer der Fig. 1 und 2, bei dem die Vorbereitungsvorrichtung entsprechend einer ersten Phase eingesetzt ist,
- Fig. 4: einen Schnitt durch einen Femurknochen gemäß einer der Fig. 1 und 2, bei dem die Vorbereitungsvorrichtung entsprechend einer zweiten Phase eingesetzt ist, die auf die erste Phase von Fig. 3 folgt.

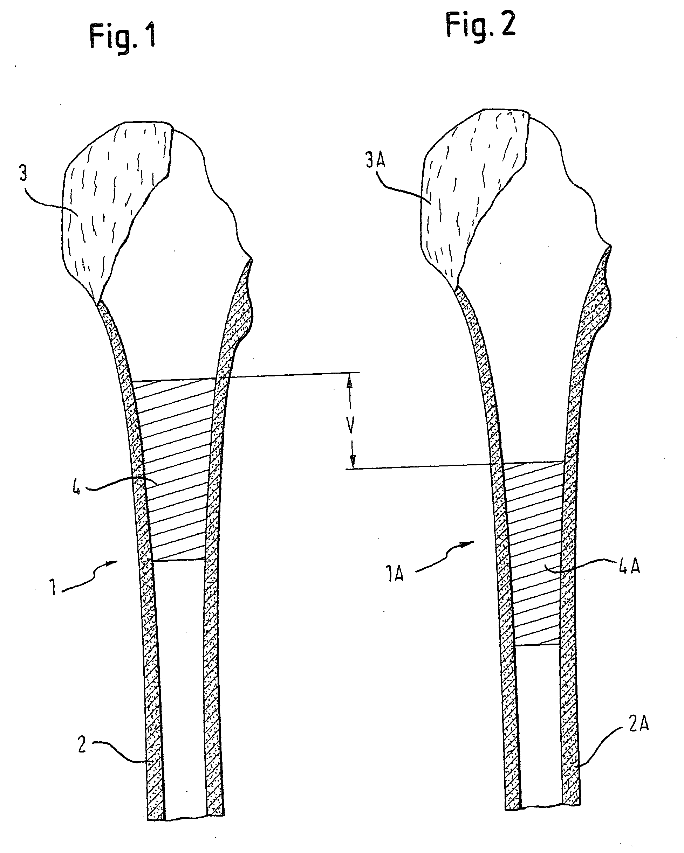

Wie die Fig. 1 und 2 zeigen, besitzt ein Femurknochen 1 oder 1A einen distalen Teil 2, 2A, der einem diaphysären Bereich des Femur 1, 1A entspricht, einen proximalen Teil 3, 3A, der einem metaphysären Bereich des Femur 1, 1A entspricht, und einen Zwischenbereich 4, 4A, in dem vorzugsweise der Press-fit-Effekt erhalten wird und der einem metaphyso-diaphysären Bereich entspricht, dessen Tiefe (Bezug Scheitel des großen Trochanter) je nach Morphotyp und Ausmaß der Knochenschäden unterschiedlich ist.

Wie in den Figuren zu sehen ist, kann die Tiefendifferenz der metaphyso-diaphysären Bereiche, in denen der Press-fit-Effekt erhalten wird, um einen Wert "V" variieren.

Genau diese Wertedifferenz muß je nach dem zu erreichenden Ziel gesteuert werden, um nicht nur den Press-fit-Effekt und damit eine gute Primärstabilität, sondern auch eine richtige Vorbereitung des metaphysären proximalen Bereichs des Femur zu erhalten, und zwar in Abhängigkeit von der Stellung und der Form des Femur proximal des genannten Press-fit-Bereichs für die Anbringung der Prothese, die der Form des Femur (Morphotyp) entspricht und deren Höhe den Erhalt einer richtigen Länge der unteren Gliedmaße gestattet, wobei ihre Primärstabilität im metaphyso-diaphysären Bereich erhalten wird und nicht im proximalen metaphysären Bereich.

Die Mittel zur konkreten Durchführung der Erfindung bestehen einerseits aus einer modulierbaren Raspel 10 aus mehreren Teilen, von denen ein stark konisches distales Teil 10A von einer einzigen Größe und von einer einzigen Form ist und von denen mindestens zwei andere proximale Teile 10B von verschiedenen Formen und aufsteigenden Höhen sind, die mit dem distalen Teil 10A durch lösbare Verbindungsmittel 11 gemäß einer in Abhängigkeit vom Morphotyp und vom Grad der Versenkung des distalen Teils bestimmten Wahl fest verbunden werden können. Diese Mittel bestehen andererseits aus einem neutralen Zwischenelement 12, das eine proximale Lehre bildet, die zur vorhergehenden Anbringung des distalen Teils 10A der Raspel 10 und zur Messung seiner Versenkung an einer Gradteilung 13 dient, die die richtige Länge des zu wiederherzustellenden Femur und damit die Größe des proximalen Teils 10B der Raspel angibt, der nacheinander zu wählen und an Ort und Stelle des Zwischenelements oder Lehre 12 zur Vorbereitung dieses Femurknochens 1 oder 1A anzuordnen ist.

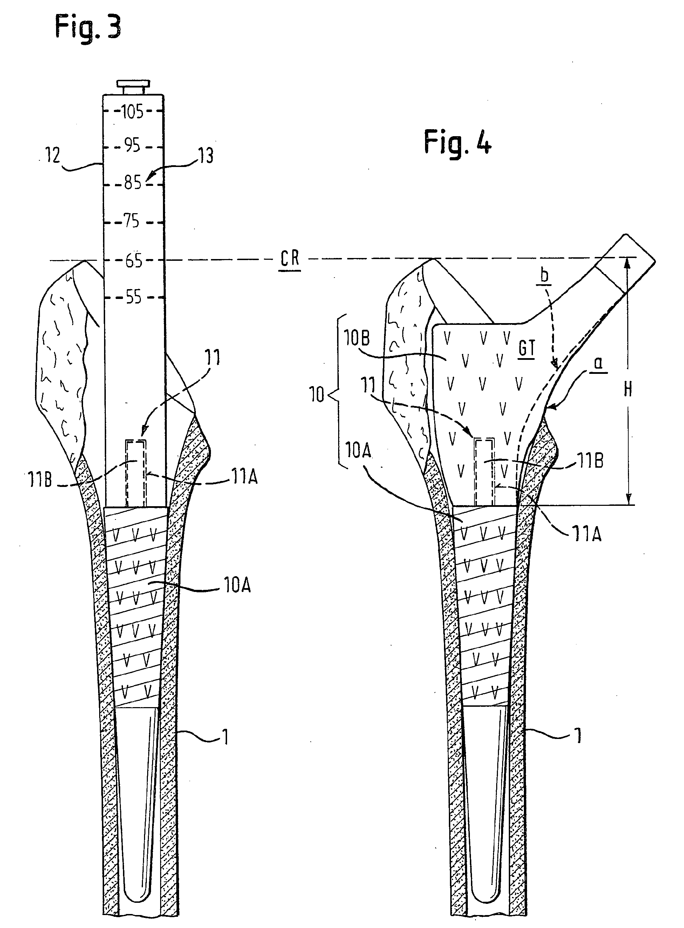

Wie Fig. 3 zeigt, ist das Zwischenelement oder Lehre 12 von zylindrischer Form mit einem Durchmesser, der etwas kleiner als der des distalen Teils 10A der Raspel ist.

Gemäß dem vorliegenden nicht begrenzenden Beispiel weist das Zwischenelement oder die Lehre 12 eine Skala 13 von sechs verschiedenen Höhen auf, die sechs proximalen Teilen 10B der Raspel 10 entsprechen.

Gemäß einem bevorzugten Ausführungsbeispiel, das in Fig. 4 dargestellt ist, sind die proximalen Teile 10B der Raspel 10 von zwei verschiedenen Formen, und jede Form gibt es mit in 10-mm-Schritten ansteigenden Höhen H von 55 mm bis 105 mm. Dabei ist die erste Form "a" die Standardform, während die zweite mögliche Form "b", die mit einer unterbrochenen Linie dargestellt ist, zylindrisch ist.

Auf dieselbe Weise hat das distale Teil 10A der Raspel 10 eine Länge von 120 mm und einen Durchmesser von 12 mm, könnte jedoch auch eine andere Länge und einen anderen Durchmesser aufweisen.

Gemäß einem anderen Merkmal der Erfindung sind die Mittel 11 zur Verbindung des proximalen Teils 10B der Raspel 10 mit ihrem distalen Teil 10A dieselben wie die desselben distalen Teils 10A der Raspel 10 und des Zwischenelements oder der Lehre 12, das bzw. die zuvor auf dieses aufgesetzt werden kann.

Gemäß dem vorliegenden Ausführungsbeispiel bestehen die Verbindungsmittel 11 aus einem Aufnahmeteil 11A, der eine abgeflachte axiale Aussparung bildet, die am Ende des proximalen Teils 10B der Raspel 10 oder am Ende des Zwischenelements oder der Lehre 12 gebildet ist und mitnehmend mit einem entsprechenden Einsteckteil 11B zusammenwirkt, der am Ende des distalen Teils 10A der Raspel 10 gebildet ist oder umgekehrt, und zwar unter einer vorbestimmten Klemmung, durch die das relative Spiel zwischen den Teilen unterdrückt werden kann.

Gemäß einer nicht dargestellten Ausführungsvariante bestehen die Verbindungsmittel aus einer Gewindebohrung, die am Ende des proximalen Teils 10B der Raspel oder am Ende des Zwischenelements oder der Lehre 12 gebildet ist und mitnehmend mit einem entsprechenden Gewindeteil zusammenwirken kann, das am Ende des distalen Teils 10A der Raspel angeordnet ist oder umgekehrt, um eine spielfreie Verbindung zu erhalten.

Das Zwischenelement 13 ist ausschließlich dazu bestimmt, das Versenken des distalen Teils 10A der modulierbaren Raspel 10 und das Messen des Rotationsmittelpunkts CR zu gestatten, um die Wahl des proximalen Teils 10B der Raspel zur Wiederherstellung der richtigen Länge der unteren Gliedmaße bei dem Setzen der Prothese zu gestatten.

Die Vorgehensweise mit dieser Vorrichtung ist die folgende:
- Sektion des Femurhalses, wenn es sich um eine Prothese für eine Erstimplantation handelt, oder Entfernung des gelockerten Implantats und des gesamten Zements, wenn es sich um einen Austausch handelt,
- seitliche Öffnung des großen Trochanter,
- Anbringen des einzigen distalen Teils 10A der Raspel 10 auf dem Zwischenelement oder Lehre 12 über die Verbindungsmittel 11,
- Eintreiben dieser Einheit in den Femurknochen 1 oder 1A bis zum Erhalten einer einwandfreien Primärstabilität der distalen Raspel, auf welcher die Lehre montiert ist, die die wirksame Ermittlung der Stabilität der Montage gestattet,
- Ablesung auf dem Griff des Zwischenelements oder Lehre 12 der Skalenteilung 13 zur Bestimmung des Grads der Versenkung, der dem Rotationsmittelpunkt CR des proximalen Teils 10B entspricht, der zu wählen ist und der mit dem Scheitel des großen Trochanter GT zusammenfällt,
- Wahl des proximalen Teils 10B der Raspel in Abhängigkeit vom Morphotyp und von der Höhe, die der vorhergehenden Ablesung auf dem Zwischenelement oder Lehre 12 entspricht,
- feste Verbindung des distalen Teils 10A mit dem ausgewählten proximalen Teil 10B. Wenn dieser nicht passend ist und nicht ausreichend weit versenkt werden kann, zeigt er dem Chirurgen an, daß er den Femur in seinem proximalen Teil aushöhlen muß, um sicher zu sein, daß die Blockierung distal bleibt,
- Ausbohren des proximalen metaphysären Teils des Femur.

Es ist zu betonen, daß diese Einheit über eine richtige Höhe versenkt werden muß, d.h. der Rotationsmittelpunkt (CR) des proximalen Teils 10B muß dem Scheitel des Trochanter (GT) entsprechen, um sicher zu sein, daß ein guter Kontakt in der metaphyso-diaphysären Zone zwischen Knochen und Implantat besteht.

Wenn dies nicht der Fall ist, müßte eine ergänzende Vorbereitung nur des proximalen metaphysären Bereichs des Femurs durchgeführt werden.

Die klinischen Versuche haben bewiesen, daß die nach der oben beschriebenen Arbeitstechnik eingesetzte erfindungsgemäße Vorrichtung eine gute Press-fit-Wirkung in dem hierfür vorgesehenen Bereich, d.h. in dem metaphyso-diaphysären Bereich, gewährleistet und gleichzeitig zur Wiederherstellung der richtigen Länge der unteren Gliedmaße führt.

## Patentansprüche

1. Vorrichtung zur Vorbereitung eines Femurknochens für die zementfreie Implantation einer Prothese mit sog. Press-fit-Effekt, die einen distalen Teil (2, 2A), der einem diaphysären Bereich des Femur (1, 1A) entspricht, einen proximalen Teil (3, 3A), der einem metaphysären Bereich des Femur entspricht, und einen Zwischenbereich (4, 4A) besitzt, in dem vorzugsweise der Press-fit-Effekt geschaffen wird und der einem metaphyso-diaphysären Bereich entspricht, dessen Höhe je nach Morphotyp und Ausmaß der Knochenschäden veränderlich ist, **dadurch gekennzeichnet, daß** sie einerseits aus einer modulierbaren Raspel (10) aus mehreren Teilen besteht, von denen ein stark konisches distales Teil (10A) von einer einzigen Größe und von einer einzigen Form ist und von denen mindestens zwei andere proximale Teile (10B) von wesentlich verschiedenen Formen und von aufsteigenden Höhen (H) sind und gemäß einer in Abhängigkeit vom Morphotyp und vom Grad der Versenkung des distalen Teils (10A) bestimmten Wahl mit dem distalen Teil (10A) durch lösbare Verbindungsmittel (11) fest verbunden werden können, und andererseits aus einem neutralen Zwischenelement (12), das eine proximale Lehre bildet, die für die vorausgehende Anbringung des distalen Teils (10A) der Raspel (10) und für die Messung seiner Versenkung an einer Gradteilung (13) bestimmt ist, die die richtige Länge des wiederherzustellenden Femur und damit die Größe des proximalen Teils (10B) der Raspel (10) angibt, das zur Vorbereitung dieses Femurknochens (1 oder 1A) zu wählen und nacheinander an Ort und Stelle des Zwischenelements (12) anzuordnen ist.

2. Vorrichtung nach Anspruch 1, **dadurch gekennzeichnet, daß** das Zwischenelement oder die Lehre (12) von zylindrischer Form ist und einen etwas kleineren Durchmesser als der distale Teil (10A) der Raspel (10) aufweist.

3. Vorrichtung nach einem der Ansprüche 1 oder 2, **dadurch gekennzeichnet, daß** das Zwischenelement oder die Lehre (12) eine Skala (13) von sechs verschiedenen Höhen besitzt, die sechs proximalen Teilen (10B) der Raspel (10) entsprechen.

4. Vorrichtung nach einem der Ansprüche 1 bis 3, **dadurch gekennzeichnet, daß** die proximalen Teile (10B) der Raspel (10) mindestens von zwei verschiedenen Formen ("a", "b") sind und jede von ansteigenden Höhen (H) ist, die in 10-mm-Schritten von 55 mm bis 105 mm gehen.

5. Vorrichtung nach einem der Ansprüche 1 bis 4, **dadurch gekennzeichnet, daß** das distale Teil (10A) der Raspel (10) eine Länge von 120 mm und einen Durchmesser von 12 mm besitzt.

6. Vorrichtung nach einem der Ansprüche 1 bis 5, **dadurch gekennzeichnet, daß** die Verbindungsmittel (11) zur Verbindung des proximalen Teils (10B) der Raspel (10) mit ihrem distalen Teil (10A) dieselben wie die desselben distalen Teils (10A) der Raspel (10) und des Zwischenelements oder Lehre (12) sind, das bzw. die zuvor auf dieses aufsetzbar ist.

7. Vorrichtung nach Anspruch 6, **dadurch gekennzeichnet, daß** die Verbindungsmittel (11) aus einem Aufnahmeteil (11A) bestehen, der eine axiale abgeflachte Aussparung bildet und am Ende des proximalen Teils (10B) der Raspel (10) oder am Ende des Zwischenelements oder der Lehre (12) gebildet ist und in mitnehmender Weise mit einem entsprechenden Einsteckteil (11B) zusammenwirken kann, der am Ende des distalen Teils (10A) der Raspel (10) gebildet ist oder umgekehrt, und zwar in einer vorbestimmten Klemmung, durch die das Relativspiel zwischen den Teilen ausgeschaltet wird.

8. Vorrichtung nach Anspruch 6, **dadurch gekennzeichnet, daß** die Verbindungsmittel aus einer Gewindebohrung bestehen, die an dem Ende des proximalen Teils (10B) der Raspel (10) oder am Ende des Zwischenelements oder der Lehre (12) gebildet ist und die in mitnehmender Weise mit einem entsprechenden mit Gewinde versehenen Teil zusammenwirken kann, der am Ende des distalen Teils (10A) der Raspel vorgesehen ist oder umgekehrt, um eine spielfreie Verbindung herzustellen.
